# FASCICULE DE BREVET EUROPEEN

(11) **EP 0 858 795 B1**
(45) Date de publication et mention de la délivrance du brevet: **04.08.2004**
(21) Numéro de dépôt: 98400050.5
(22) Date de dépôt: 13.01.1998
(51) Int. Cl.: A61K 7/11, A61K 7/48

(54) **Utilisation de polymères présentant une température critique du type LCST ou du type UCST dans et pour la fabrication de formulations cosmétiques ou dermatologique, compositions les contenant**
Verwendung von Polymer mit einer kritischer Temperatur von der LCST oder UCST-Type in und für die Herstellung von einer kosmetischen oder dermatologischen Zusammensetzung, sowie Zusammensetzungen diese enthaltend
Use of polymers having a critical temperature of the LCST or UCST-type in and for the preparation of dermo-cosmetic compositions, and compositions containing these

(30) Priorité: 10.02.1997 FR 9701499
(43) Date de publication de la demande: 19.08.1998
(73) Titulaire: L'OREAL, 75008 Paris (FR)
(72) Inventeur: Mougin, Nathalie, 75011 Paris (FR)
(74) Mandataire: Dodin, Catherine

(56) Documents cités:
- EP-A- 0 455 081
- WO-A-93/07856
- WO-A-93/11736
- WO-A-95/01383
- WO-A-96/36310
- DE-A- 3 731 477
- DATABASE WPI Week 9138 Derwent Publications Ltd., London, GB; AN 91-278280 XP002048385 & JP 03 184 910 A (SANSHO KK) 12 août 1991

## Description

La présente invention concerne des compositions cosmétiques ou dermatologiques contenant des polymères non-réticulés susceptibles de former, après séchage, un dépôt ou un film sur un support kératinique et présentant une température critique Tc de solubilité dans l'eau du type LCST ou UCST ainsi que leurs diverses applications en particulier dans le domaine des produits capillaires et dans le domaine des produits de maquillage.

On entendra dans toute la description par produit cosmétique ou dermatologique «non-rincé», tout produit dont l'application sur les matières kératiniques à traiter n'est pas suivie d'un rinçage à l'eau.

On entendra dans toute la description par produit cosmétique ou dermatologique «rincé», tout produit dont l'application sur les matières kératiniques à traiter est suivie d'un rinçage à l'eau.

On entendra dans toute la description par matières kératiniques, les matières à traiter cosmétiquement ou dermatologiquement choisies parmi la peau, le cuir chevelu, les cheveux, les cils, les sourcils, les ongles ou les muqueuses.

De nombreuses applications cosmétiques non-rincées font appel au transport d'un polymère à partir d'une solution organique, aqueuse ou hydroorganique devant donner, après séchage et évaporation de la solution, sur la matière kératinique à traiter un dépôt ou un film présentant des caractéristiques mécaniques spécifiques à l'utilisation envisagée. C'est le cas, par exemple, des produits de coiffage tels que les laques capillaires contenant en général des alcools ou bien des produits de maquillage comme les vernis à ongles contenant en général des solvants du type acétate de vinyle.

Lorsque le polymère est soluble dans un solvant organique, il possède en général une bonne affinité avec celui-ci. Pendant la phase de séchage et d'évaporation dudit solvant, après application sur le support kératinique, le polymère retient le plus souvent un résidu dudit solvant qui a tendance d'une part à affecter substantiellement les propriétés mécaniques recherchées du film ou du dépôt, en le plastifiant temporairement sur une durée assez longue et en diminuant sensiblement sa rigidité et d'autre part à prolonger sensiblement le temps de séchage, ce qui rend difficile l'application et nuit au confort de l'utilisateur. Dans le cadre des produits de coiffage et/ou de maintien des cheveux, on obtient généralement dans de telles conditions, des films ou des dépôts difficilement démêlables et dans le cadre des vernis à ongles ou des bases de soin des ongles, on obtient des films trop mous voire collants au toucher et évolutifs dans le temps.

Lorsque le polymère est dissous dans une solution aqueuse ou hydroorganique, le film ou le dépôt obtenu après séchage et évaporation de la solution, en plus des inconvénients énoncés ci-dessus aura tendance à être très sensible à l'humidité ambiante et peu résistant à l'eau, ce qui est particulièrement gênant dans les produits de coiffage et les produits de maquillage où l'on recherche une bonne rémanence à l'eau.

Pour pallier à ces différents problèmes, on a proposé d'utiliser à la place des solutions organiques, aqueuses ou hydro-organiques de polymère filmogène, des dispersions aqueuses de particules de polymère filmogène du type latex ou pseudo-latex. Dans le cadre des produits de coiffage et/ou de maintien des cheveux et plus particulièrement des laques capillaires, pour obtenir un film ou un dépôt sur les cheveux présentant des caractéristiques mécaniques et cosmétiques satisfaisantes, il est nécessaire d'utiliser lesdits polymères à des concentrations en extrait sec élevées (en général de l'ordre de 15 à 20% en poids). On obtient alors des temps de séchage beaucoup trop longs. En augmentant l'extrait sec en latex ou pseudo-latex, on réduit sensiblement le temps de séchage mais on obtient un effet de «cartonnage» des cheveux, peu désirable sur le plan cosmétique, dû à un pouvoir fixant trop important et des difficultés importantes de démêlage et d'élimination au shampooing apparaissent.

Un des objectifs de la présente invention est de rechercher, dans le cadre des produits cosmétiques non-rincés, des nouvelles solutions de polymère permettant, dans des temps de séchage sensiblement plus courts, d'obtenir un film ou un dépôt présentant des propriétés mécaniques et cosmétiques satisfaisantes et non-évolutives dans l'application cosmétique envisagée, en réduisant au maximum la quantité de solvant piégé dans le film ou dépôt en fin de séchage.

De nombreuses applications cosmétiques rincées font appel à l'utilisation d'un polymère en solution ou en dispersion dans un milieu aqueux devant donner, après application sur la matière kératinique à traiter et rincage à l'eau, un dépôt. C'est le cas, par exemple, des shampooings coiffants ou conditionneurs contenant généralement une forte concentration de tensio-actifs et un polymère susceptible de se déposer sur les cheveux pour apporter un effet de maintien ou un effet conditionneur permettant d'améliorer certaines propriétés cosmétiques comme le démêlage, le toucher, la brillance, la souplesse. C'est également le cas des produits d'hygiène pour la peau tels que les formulations pour le bain ou la douche ou les démaquillants. Cependant, après application et l'étape de rinçage à l'eau, la majorité du polymère est généralement éliminé et le dépôt formé sur les cheveux ou la peau est trop faible voire inexistant pour apporter de manière efficace les propriétés cosmétiques souhaitées.

Un autre objectif de la présente invention est donc de rechercher, dans le cadre des produits cosmétiques rincés, des nouvelles solutions ou dispersions aqueuses de polymère permettant d'obtenir sur la matière kératinique à traiter, après application et rinçage à l'eau, une quantité de dépôt de polymère suffisante pour apporter les propriétés cosmétiques recherchées selon l'application envisagée.

On connaît dans l'état de la technique des polymères particuliers dont la solubilité dans l'eau est modifiée au-delà d'une certaine température. Il s'agit des polymères présentant une température critique (ou point de trouble) définissant leur zone de solubilité dans l'eau. Cette température est appelée « LCST » (Lower Critical Solution Temperature) lorsqu'au dessus de cette température, le polymère perd sa solubilité dans l'eau et devient soluble dans l'eau en dessous de cette température critique. Cette température est appelée « UCST » (Upper Critical Solution Temperature) lorsqu'en dessous de cette température, le polymère perd sa solubilité dans l'eau et devient soluble dans l'eau au dessus de cette température critique. Ces polymères sont notamment décrits dans l'article de A.S. HOFFMAN Macromol. Symp., 98, pp 645-664 (1995) ainsi que dans l'ouvrage «Comprehensive Polymer Science, pp 122-124, 1989-PERGAMON PRESS». Selon l'enseignement des demandes de brevet EP-A-0583814 et EP-A-0629649, certains polymères ayant une température critique du type LCST sont utilisés comme additifs viscosifiants thermoréversibles dans la fabrication de fluides ou de lubrifiants utilisés dans de nombreux secteurs industriels.

La Demanderesse a découvert de manière surprenante que certains polymères non-réticulés ayant une température critique Tc de solubilité dans l'eau du type LCST ou UCST allant de 0° à 100°C, associés à un système solvant hydroorganique approprié, produisaient après application sur des matières kératiniques et séchage dudit système solvant, un film ou un dépôt présentant des propriétés mécaniques et cosmétiques satisfaisantes pour apporter les propriétés cosmétiques recherchées selon l'application envisagée ; les temps de séchage étant sensiblement plus courts que ceux des solutions de polymère filmogène habituellement utilisées dans les produits cosmétiques non-rincés et les taux de solvant résiduel piégé dans le film ou le dépôt obtenu en fin de séchage sont substantiellement plus faibles voire inexistants.

La Demanderesse a découvert également que ces mêmes polymères particuliers associés à un milieu aqueux approprié contenant des tensio-actifs et/ou certains polymères hydrophiles susceptibles d'établir avec lesdits polymères de l'invention des interactions physiques, permettaient d'obtenir, après application sur des matières kératiniques et rincage à l'eau, une quantité de dépôt de polymère suffisante pour apporter les propriétés cosmétiques recherchées selon l'application envisagée.

Un objet de l'invention concerne donc l'utilisation comme agent de revêtement des matières kératiniques, dans et pour la préparation de compositions cosmétiques ou dermatologiques, d'une solution hydroorganique comportant au moins :
(a) un polymère non-réticulé susceptible de former un dépôt ou un film sur un support kératinique après séchage et présentant une température critique Tc de solubilité dans l'eau du type LCST ou UCST allant de 0° à 100°C ;
(b) au moins un solvant organique total dudit polymère dans le domaine de température d'utilisation de la composition, miscible partiellement ou totalement à l'eau et plus volatil que l'eau.

L'invention concerne également des compositions cosmétiques ou dermatologiques, caractérisées par le fait qu'elles contiennent au moins, comme agent de revêtement des matières kératiniques, une solution hydroorganique comportant:
(a) au moins un polymère non-réticulé susceptible de former un dépôt ou un film sur un support kératinique après séchage et présentant une température critique Tc de solubilité dans l'eau du type LCST ou UCST allant de 0° à 100°C ;
(b) au moins un solvant organique total dudit polymère dans le domaine de température d'utilisation de la composition, miscible partiellement ou totalement à l'eau et plus volatil que l'eau. Ce type de composition est tout particulièrement adapté aux applications non-rincées.

Un autre objet de l'invention concerne l'utilisation comme agent de revêtement des matières kératiniques, dans et pour la préparation de compositions cosmétiques ou dermatologiques, d'une solution aqueuse ou d'une dispersion aqueuse comportant:
(a) au moins un polymère non-réticulé susceptible de former un dépôt sur un support kératinique après séchage et présentant une température critique Tc de solubilité dans l'eau du type LCST ou UCST allant de 0° à 100°C ;
(b) au moins un agent tensio-actif et/ou au moins un polymère hydrophile ne présentant pas de température critique Tc comprise dans le domaine indiqué ci-dessus, susceptibles d'établir avec ledit polymère des interactions physiques.

L'invention concerne également des compositions cosmétiques ou dermatologiques, caractérisées par le fait qu'elles contiennent, comme agent de revêtement des matières kératiniques, au moins une solution aqueuse ou une dispersion aqueuse comportant :
(a) au moins un polymère susceptible de former, après séchage et évaporation, un dépôt ou un film sur un support kératinique et présentant une température critique Tc de solubilité dans l'eau du type LCST ou du type UCST allant de 0° à 100°C;
(b) au moins un agent tensio-actif et/ou au moins un polymère hydrophile ne présentant pas de température critique Tc comprise dans le domaine indiqué ci-dessus, susceptibles d'établir avec ledit polymère des interactions physiques, comme agent de revêtement des matières kératiniques. Ce type de composition est tout particulièrement adapté aux produits rincés.

D'autres objets apparaitront à la lumière de la description et des exemples qui suivent.

La température d'utilisation des compositions de l'invention varie en général de 0 à 100°C et plus particulièrement correspond à la température ambiante.

La température critique Tc de solubilité dans l'eau varie de 0° à 100°C et plus préférentiellement de 10 à 80°C.

Les polymères selon l'invention, présentant une température critique Tc dans le domaine indiqué ci-dessus du type LCST sont choisis dans le groupe constitué par :
(i) les homopolymères ou copolymères non-réticulés de monomères comportant un groupe amide comme les poly(acrylamide N-substitué) tels que les homopolymères poly(N-isopropylacrylamide), les copolymères de N-isopropylacrylamide et d'ester d'acide (méth)acrylique en C₁-C₁₈ (par exemple acrylate de butyle), les copolymères de N-isopropylacrylamide et d'acide méthacrylique

Les polymères selon l'invention présentant une température critique de solubilité dans l'eau du type LCST ou UCST sont présents dans les compositions conformes à l'invention dans des concentrations allant de préférence de 0,1 à 50% en poids et plus préférentiellement de 0,5 à 40% en poids par rapport au poids total de la composition. La concentration en polymère dépendra de l'application cosmétique ou dermatologique envisagée.

Les solvants organiques utilisés dans les compositions de l'invention sont choisis parmi tous les solvants organiques cosmétiquement acceptables susceptibles de dissoudre totalement les polymères de l'invention dans le domaine de température d'utilisation. Ils doivent être miscibles partiellement ou totalement dans l'eau et plus volatils que l'eau.

Ils peuvent être choisis par exemple parmi :
- les alcools inférieurs en C₁-C₄ tels que l'éthanol, l'isopropanol, le n-propanol ;
- les éthers tels que le diméthoxyéthane ;
- les cétones telles que l'acétone, le méthyléthylcétone ;
- les esters d'acide carboxylique inférieurs en C₁-C₃ tels que l'acétate de méthyle, l'acétate d'éthyle.

Les solvants organiques de l'invention sont présents dans les compositions conformes à l'invention dans des concentrations allant de 5 à 90% en poids et plus préférentiellement de 10 à 70% en poids par rapport au poids total du mélange eau/solvant(s) organique(s).

Une forme particulière de l'invention consiste en des compositions contenant au moins un tensio-actif et/ou au moins un polymère hydrophile (soluble ou dispersible dans l'eau) additionnel ne présentant pas une température critique du type LCST ou UCST dans le domaine préconisé par l'invention, susceptibles d'établir avec les polymères de l'invention des interactions physiques.

Les interactions physiques peuvent être du type ionique, polaire, dipolaire, hydrophobe, hydrogène.

Parmi les polymères hydrophiles additionnels susceptibles d'interagir avec les polymères de l'invention et ne présentant pas une température critique du type LCST ou UCST dans le domaine préconisé par l'invention, on peut citer par exemple :
- les alcools polyvinyliques ainsi que leurs copolymères (en particulier avec l'acétate de vinyle ou l'éthylène) ;
- les polysaccharides ou les polymères cellulosiques tels que les éthers de cellulose hydrosolubles, les carboxyméthylcelluloses, les celluloses cationiques, les gommes de guar hydroxypropylées, les gommes de guar comportant à la fois des groupes hydroxypropyle et des groupes ioniques tels que carboxyméthyle, chlorure de triméthylammonium, les gommes de xanthane, le gellane, le chitosane, l'acide hyaluronique;
- les protéines naturelles ou les polypeptides synthétiques ;
- les polymères synthétiques de monomères à groupes amides comme les homopolymères ou copolymères de vinylpyrrolidone ;
- les copolymères à base de monomères à groupes anioniques tels que carboxylique, sulfonique ou phosphonique comme par exemple les poly(acide acrylamido sulfonique) et les polymères poly(acide vinyl phosphonique).

Ces polymères hydrophiles additionnels sont présents dans les compositions conformes à l'invention dans des concentrations allant de préférence de 5 à50% en poids et plus préférentiellement de 10 à 30% en poids par rapport au poids total de tous les polymères présents dans la composition.

Le ou les tensioactifs associés aux polymères ayant une température critique du type LCST ou UCST dans le domaine indiqué ci-dessus peuvent être indifféremment choisis, seuls ou en mélanges, au sein des tensioactifs anioniques, amphotères, non ioniques, zwittérioniques et cationiques.

Ainsi, selon l'invention, les tensioactifs représentent de 4 % à 30 % en poids, plus préférentiellement de 10 % à 25 % en poids, et encore plus préférentiellement de 12 % à 20 % en poids, du poids total de la composition finale.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylaryl polyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfo-succinates, les alkyfamidesulfosuccinates ; les alkylsulfosuccinamates ; les alkylsulfo-acétates ; les alkyl éther phosphates ; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (I) suivante :

R₁―(-OC₂H₄-)ₙ―OCH₂COOA (1)

dans laquelle :
R₁ désigne un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A désigne H, ammonium, Na, K, Li, Mg ou un reste monoéthanolamine ou triéthanol-amine. On peut également utiliser des mélanges de composés de formule (1) en particulier des mélanges dans lesquels les groupements R₁ sont différents.

Des composés de formule (1) sont vendus par exemple par la Société CHEM Y sous les dénominations AKYPOS (NP40, NP70, OP40, OP80, RLM25, RLM38, RLMQ38 NV, RLM 45, RLM 45 NV, RLM 100, RLM 100 NV, RO20, RO 90, RCS 60, RS 60, RS 100, RO 50) ou par la Société SANDOZ sous les dénominations SANDOPAN (DTC Acid, DTC).

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras de polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s) ou zwittérionique(s) :

Les agents tensioactifs amphotères ou zwittérioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2528378 et US-2781354 et classés dans le dictionnaire CTFA, 3ème édition, 1982, sous les dénominations Amphocarboxy-glycinates et Amphocarboxypropionates de structures respectives :

R₂ -CONHCH₂CH₂ -N(R₃)(R₄)(CH₂COO-) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂-CONHCH₂CH₂-N(B)(C) (3)

dans laquelle :
B représente -CH₂CH₂OX', C représente -(CH₂)_{Z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂ désigne un radical alkyle d'un acide R₉ -COOH présent dans l'huile de coprah
ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate vendu sous la dénomination commerciale MIRANOL C₂M concentré par la Société MIRANOL.

### (iv) Tensioactifs cationiques :

Parmi les tensioactifs cationiques, dont la nature ne revêt pas dans le cadre de la présente invention de caractère critique, on peut citer en particulier (liste non limitative) : les sels d'amines grasses primaires, secondaires ou tertiaires, éventuellement polyoxy-alkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline; ou les oxydes d'amines à caractère cationique.

On notera que les tensioactifs cationiques, dont l'utilisation n'est pas exclue, ne constituent pas des tensioactifs préférés pour la mise en oeuvre de la présente invention.

De façon connue, toutes les compositions de l'invention peuvent contenir des adjuvants habituels dans les domaines cosmétique et dermatologique, des huiles, cires ou autres corps gras usuels, des gélifiants et/ou épaississants classiques ; des émulsionnants ; des agents hydratants ; des émollients ; des filtres solaires ; des actifs hydrophiles ou lipophiles comme des céramides ; des agents anti-radicaux libres ; des bactéricides ; des séquestrants; des antipelliculaires ; des antioxydants ; des conservateurs ; des agents alcanisants ou acidifiants ; des parfums ; des charges ; des matières colorantes. Les quantités de ces différents adjuvants sont celles classiquement utilisées dans les domaines considérés.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous toutes les formes appropriées pour une application topique, notamment sous forme de solutions du type lotion ou sérum ; sous forme de gels aqueux; sous forme d'émulsions obtenues par dispersion d'une phase grasse dans une phase aqueuse (H/E) ou inversement (E/H), de consistance liquide plus ou moins épaissie telles que des laits, des crèmes plus ou moins onctueuses. Ces compositions sont préparées selon les méthodes usuelles.

Les compositions selon l'invention peuvent être utilisées comme produits capillaires notamment pour le lavage, le soin, le conditionnement, le maintien de la coiffure ou la mise en forme des fibres kératiniques telles que les cheveux.

Les compositions non-rincées capillaires selon invention sont de préférence des produits de coiffage tels que des lotions de mise en plis, des lotions pour le brushing, des compositions de fixation et de coiffage telles que des laques ou spray. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une mousse pour la fixation ou le traitement des cheveux.

Les compositions capillaires rincées sont plus particulièrement des shampooings coiffants et/ou conditionneurs ou bien des après-shampooings à rincer.

Les compositions de l'invention peuvent être également utilisées comme produit de soin et/ou l'hygiène tels que des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions de l'invention peuvent être également utilisées comme démaquillants.
Les compositions peuvent être des produits pour le maquillage et plus particulièrement des vernis à ongles ou des bases de soin pour les ongles.

Un autre objet de l'invention est un procédé de traitement non-thérapeutique cosmétique de la peau, du cuir chevelu, des cheveux, des cils, des sourcils, des ongles ou des muqueuses, caractérisé par le fait qu'on applique sur le support kératinique une composition telle que définie ci-dessus, selon la technique d'utilisation habituelle de cette composition. Les exemples suivants illustrent l'invention sans présenter un caractère limitatif.

Les exemples qui suivent servent à illustrer la présente invention sans toutefois présenter un caractère limitatif.

### EXEMPLES

### EXEMPLE 1 : Synthèse de l'homopoly(N-isopropyl-acrylamide)

On purifie le monomère N-isopropylacrylamide par dissolution dans l'acétone et recristallisation dans l'éther de pétrole.

Dans un réacteur muni d'une agitation centrale, d'un thermomètre et d'une arrivée d'azote, on introduit :
- N-isopropylacrylamide 50 g
- Méthyléthylcétone (solvant) 125 g
- T-butyl-2-éthyl-hexanoate vendu sous le nom TRIGONOX par AKZO 2 g

On réalise la polymérisation sous azote et agitation pendant 18 heures à 79°C. On précipite le polymère dans l'éther éthylique puis on filtre et on lave au diéthyléther. Le polymère est séché , sous vide, à 40°C durant 24heures.

On mesure ensuite la température critique Tc de solubilité du type LCST de ce polymère par méthode visuelle et par DSC au moyen d'un appareil du type «ROBOTEC SYSTEM DSC 7 RS» vendu par la société PERKIN ELMER. Selon la méthode visuelle, on réalise une solution dans l'eau dudit polymère à une concentration donnée puis on la place dans un flacon. Ce dernier est placé dans un bain thermostaté. Lorsque la Tc à déterminer est supérieure à 20°C, la température du bain est augmentée progressivement de 0,5 en 0,5°C avec un palier de 10 minutes à chaque température. Lorsque la Tc à déterminer est inférieure à 20°C, le flacon est placé dans un bain thermostaté dont la température est régulé par un cryostat et on abaisse progressivement de 0,5 en 0,5°C avec un palier de 10 minutes à chaque température. La température à laquelle commence le trouble est déterminée visuellement.

Selon la méthode DSC, la valeur de la température Tc est déterminée sur la première montée en température de l'échantillon avec une vitesse de montée en température de 0,1°C/minute. La valeur de la transition est calculée au maximum du pic.

Le polymère obtenu présente une température critique du type LCST égale à environ 31°C, dans une solution dans l'eau à 5% en poids.

### EXEMPLE 2: Synthèse d'un copolymère de N-isopropyl-acrylamide et d'acrylate de butyle

On opère dans les mêmes conditions que celles mises en oeuvre dans l'exemple 1 en utilisant un mélange de monomères constitué de :
- N-isopropylacrylamide 90% en poids
- Acrylate de butyle 10% en poids

Le copolymère obtenu présente une température critique du type LCST égale à environ 19 °C, dans une solution dans l'eau à 5% en poids.

### EXEMPLE 3: Synthèse d'un copolymère de N-isopropyl-acrylamide et d'acide acrylique

On opère dans les mêmes conditions que celles mises en oeuvre dans l'exemple 1 en utilisant un mélange de monomères constitué de :
- N-isopropylacrylamide 90% en poids
- Acide acrylique 10% en poids

Le copolymère obtenu présente une température critique du type LCST égale à environ 30,7 °C, dans une solution dans l'eau à 5% en poids.

### EXEMPLE 4 : Synthèse d'un copolymère de N-isopropyl-acrylamide et de méthacrylate de diméthylaminoéthyle

On opère dans les mêmes conditions que celles mises en oeuvre dans l'exemple 1 en utilisant un mélange de monomères constitué de :
- N-isopropylacrylamide 90% en poids
- Méthacrylate de diméthylaminoéthyle 10% en poids

Le copolymère obtenu présente une température critique du type LCST égale à environ 29 °C, dans une solution dans l'eau à 5% en poids.

### EXEMPLE 5 : Laque aérosol capillaire hydroalcoolique pour la fixation des cheveux

On réalise une laque aérosol en dissolvant 7 g de polymère selon l'exemple 1 dans un mélange constitué de 27,9 g d'éthanol et 18,6g d'eau (mélange éthanol/eau ; 60/40 % en poids). La solution limpide obtenue est introduite dans un dispositif aérosol contenant 50 g de propulseur diméthyléther.

On obtient ainsi une laque capillaire qui, après séchage très rapide (à un temps équivalent de celui d'une laque alcoolique), produit un dépôt sur les cheveux apportant une bonne fixation suffisamment rigide sans effet collant au toucher. Le dépôt se démêle facilement au passage du peigne ou de la brosse.

### EXEMPLE 6 : Laque aérosol capillaire hydroalcoolique pour la fixation des cheveux

On réalise une laque aérosol en dissolvant 7 g de polymère selon l'exemple 2 dans un mélange constitué de 27,9 g d'éthanol et 18,6g d'eau (mélange ethanol/eau ; 60/40 % en poids). La solution limpide obtenue est introduite dans un dispositif aérosol contenant 50 g de propulseur diméthyléther.

On obtient également une laque capillaire qui, après séchage très rapide malgré la quantité importante d'eau (à un temps équivalent de celui d'une laque alcoolique), produit un dépôt sur les cheveux apportant une bonne fixation suffisamment rigide sans effet collant au toucher. Le dépôt se démêle facilement au passage du peigne ou de la brosse.

## Revendications

1. Utilisation comme agent de revêtement des matières kératiniques dans une composition cosmétique, d'une solution hydroorganique comportant:
(a) 0,1 à 50% en poids par rapport au poids total de la composition, d'au moins un polymère non-réticulé susceptible de former un dépôt ou un film sur un support kératinique après séchage et présentant une température critique Tc de solubilité dans l'eau du type LCST allant de 0° à 100°C, choisi dans le groupe constitué par les homopolymères ou copolymères non-réticulés de monomères comportant un groupe amide choisis parmi les poly(acrylamide N-substitué) tels que les homopolymères poly(N-isopropylacrylamide), les copolymères de N-isopropylacrylamide et d'ester d'acide (méth)acrylique en C₁-C₁₈ (par exemple acrylate de butyle), les copolymères de N-isopropylacrylamide et d'acide méthacrylique;
et
(b) 5 à 90% en poids par rapport au poids total du mélange eau/solvant(s), d'au moins un solvant organique total dudit polymère dans le domaine de température d'utilisation de la composition, miscible partiellement ou totalement à l'eau et plus volatil que l'eau.

2. Utilisation selon la revendication 1, dans laquelle le solvant organique est choisi parmi:
- les alcools inférieurs en C₁-C₄ tels que l'éthanol, l'isopropanol, le n-propanol;
- les éthers tels que le diméthoxyéthane;
- les cétones telles que l'acétone, le méthyléthylcétone;
- les esters d'acide carboxylique inférieurs en C₁-C₃ tels que l'acétate de méthyle, l'acétate d'éthyle.

3. Utilisation comme agent de revêtement des matières kératiniques dans une composition cosmétique, d'une solution aqueuse ou d'une dispersion aqueuse comportant:
(a) 0,1 à 50% en poids par rapport au poids total de la composition, d'au moins un polymère non-réticulé susceptible de former, dans le domaine de température d'utilisation de la composition, un dépôt sur un support kératinique après séchage et présentant une température critique Tc de solubilité dans l'eau du type LCST allant de 0° à 100°C, choisi dans le groupe constitué par les homopolymères ou copolymères non-réticulés de monomères comportant un groupe amide choisis parmi les homopolymères de poly(N-isopropylacrylamide), les copolymères de N-isopropyl-acrylamide et d'ester d'acide (méth)acrylique en C₁-C₁₈ (par exemple acrylate de butyle), les copolymères de N-isopropylacrylamide et d'acide méthacrylique; les polymères ou copolymères de vinylcaprolactame;
et
(b) au moins un agent tensioactif représentant 4 à 30% en poids, préférentiellement de 10 à 25% en poids, plus préférentiellement de 12 à 20% en poids, du poids total de la composition finale, et/ou au moins un polymère hydrophile ne présentant pas de température critique Tc comprise dans le domaine indiqué ci-dessus, susceptible d'établir avec ledit polymère des interactions physiques, présent dans la composition à une concentration allant de 5 à 50% en poids, préférentiellement de 10 à 30% en poids, par rapport au poids total de tous les polymères présents dans la composition.

4. Utilisation selon la revendication 3, dans laquelle le polymère hydrophile est choisi dans le groupe constitué par:
- les alcools polyvinyliques ainsi que leurs copolymères;
- les polysaccharides ou les polymères cellulosiques;
- les protéines naturelles ou les polypeptides synthétiques;
- les polymères synthétiques de monomères à groupes amides;
- les copolymères à base de monomères à groupes anioniques de préférence carboxylique, sulfonique ou phosphonique.

5. Utilisation selon la revendication 3, dans laquelle l'agent tensioactif est choisi, seul ou en mélange, au sein des tensioactifs anioniques, amphotères, non ioniques, zwittérioniques et cationiques.

6. Composition cosmétique ou dermatologique, **caractérisée par le fait qu'**elle contient au moins, comme agent de revêtement des matières kératiniques, une solution hydroorganique comportant:
(a) 0,1 à 50% en poids par rapport au poids total de la composition, d'au moins un polymère non-réticulé susceptible de former un dépôt ou un film sur un support kératinique après séchage et présentant une température critique Tc de solubilité dans l'eau du type LCST allant de 0° à 100°C, choisi dans le groupe constitué par les homopolymères ou copolymères non-réticulés de monomères comportant un groupe amide choisis parmi les poly(acrylamide N-substitué) tels que leshomopolymères poly(N-isopropylacrylamide), les copolymères de N-isopropylacrylamide et d'ester d'acide (méth)acrylique en C₁-C₁₈ (par exemple acrylate de butyle), les copolymères de N-isopropylacrylamide et d'acide méthacrylique ;
et
(b) 5 à 90% en poids par rapport au poids total du mélange eau/solvant(s), d'au moins un solvant organique total dudit polymère dans le domaine de température d'utilisation de la composition, miscible partiellement ou totalement à l'eau et plus volatil que l'eau.

7. Composition selon la revendication 6, dans laquelle les solvants organiques sont présents dans des concentrations allant de 10 à 70% en poids par rapport au poids total du mélange eau/solvant(s) organique(s).

8. Composition selon l'une des revendications 6 à 7, dans laquelle le solvant organique est choisi parmi:
- les alcools inférieurs en C₁-C₄ tels que l'éthanol, l'isopropanol, le n-propanol ;
- les éthers tels que le diméthoxyéthane ;
- les cétones telles que l'acétone, le méthyléthylcétone ;
- les esters d'acide carboxylique inférieurs en C₁-C₃ tels que l'acétate de méthyle, l'acétate d'éthyle.

9. Composition cosmétique ou dermatologique, **caractérisée par le fait qu'**elle contient au moins, comme agent de revêtement des matières kératiniques, une solution aqueuse ou une dispersion aqueuse comportant :
(a) 0,1 à 50% en poids par rapport au poids total de la composition, d'au moins un polymère non-réticulé susceptible de former, dans le domaine de température d'utilisation de la composition, un dépôt sur un support kératinique après séchage et présentant une température critique Tc de solubilité dans l'eau du type LCST allant de 0° à 100°C choisi dans le groupe constitué par les homopolymères ou copolymères non-réticulés de monomères comportant un groupe amide choisis parmi les homopolymères de poly(N-isopropylacrylamide), les copolymères de N-isopropyl-acrylamide et d'ester d'acide (méth)acrylique en C₁-C₁₈ (par exemple acrylate de butyle), les copolymères de N-isopropylacrylamide et d'acide méthacrylique; les polymères ou copolymères de vinylcaprolactame;
et
(b) au moins un agent tensioactif représentant 4 à 30% en poids, préférentiellement de 10 à 25% en poids, plus préférentiellement de 12 à 20% en poids, du poids total de la composition finale, et/ou au moins un polymère hydrophile ne présentant pas de température critique Tc comprise dans le domaine indiqué ci-dessus, susceptible d'établir avec ledit polymère des interactions physiques, présent dans la composition à une concentration allant de 5 à 50% en poids, préférentiellement de 10 à 30% en poids, par rapport au poids total de tous les polymères présents dans la composition.

10. Composition selon la revendication 9, dans laquelle dans laquelle le polymère hydrophile est choisi dans le groupe constitué par:
- les alcools polyvinyliques ainsi que leurs copolymères;
- les polysaccharides ou les polymères cellulosiques;
- les protéines naturelles ou les polypeptides synthétiques;
- les polymères synthétiques de monomères à groupes amides;
- les copolymères à base de monomères à groupes anioniques de préférence carboxylique, sulfonique ou phosphonique.

11. Composition selon l'une des revendications 6 à 10, dans laquelle les polymères non réticulés sont présents dans des concentrations allant de 0,5 à 40% en poids, par rapport au poids total de la composition.

12. Composition selon l'une des revendications 6 à 11, **caractérisée par le fait qu'**elle est utilisée comme produit capillaire, notamment pour le lavage, le soin, le conditionnement, le maintien de la coiffure ou la mise en forme des fibres kératiniques.

13. Composition selon l'une des revendications 6 à 12, **caractérisée par le fait qu'**elle est utilisée comme composition non-rincée capillaire, notamment comme produits de coiffage tels que lotions de mise en plis, lotions pour le brushing, compositions de fixation et de coiffage telles que des laques ou sprays.

14. Composition selon l'une des revendications 6 à 12, **caractérisée par le fait qu'**elle est utilisée comme composition capillaire rincée, en particulier comme shampooing coiffant et/ou conditionneur, ou comme après-shampooing.

15. Composition selon l'une quelconque des revendications 6 à 11, **caractérisée par le fait qu'**elle est utilisée comme produit de soin et/ou d'hygiène.

16. Composition selon l'une quelconque des revendications 6 à 11, **caractérisée par le fait qu'**elle est utilisée comme démaquillant.

17. Composition selon l'une quelconque des revendications 6 à 11, **caractérisée par le fait qu'**elle est utilisée comme produit pour le maquillage.

18. Composition selon la revendication 17, **caractérisée par le fait qu'**elle est utilisée comme vernis à ongles ou base de soin pour les ongles.

19. Procédé de traitement non-thérapeutique cosmétique de la peau, du cuir chevelu, des cheveux, des cils, des sourcils, des ongles ou des muqueuses, **caractérisé par le fait qu'**on applique sur le support kératinique une composition telle que définie selon l'une quelconque des revendications 6 à 18, selon la technique d'utilisation habituelle de cette composition.

## Patentansprüche

1. Verwendung einer wässerig-organischen Lösung als Mittel zum Überziehen keratinischer Materialien in einer kosmetischen Zusammensetzung, wobei die wässerig-organische Lösung enthält:
(a) 0,1 bis 50 Gew.-%, bezogen auf das Gesamtgewicht der Zusammensetzung, mindestens eines nicht vernetzten Polymers, das nach dem Trocknen zur Bildung einer Abscheidung oder eines Films auf einem keratinischen Träger befähigt ist und eine kritische Lösungstemperatur Tk in Wasser vom Typ LCST im Bereich von 0 bis 100°C aufweist und das unter den nicht vernetzten Homopolymeren oder Copolymeren von Monomeren, die eine Amidgruppe aufweisen, ausgewählt ist, die unter den Poly(N-substituierten Acrylamiden), wie den Poly(N-isopropylacrylamid)-Homopolymeren, den Copolymeren von N-Isopropylacrylamid und einem C₁₋₁₈-(Meth)acrylsäureester (beispielsweise Butylacrylat) und den Copolymeren von N-Isopropylacrylamid und Methacrylsäure ausgewählt sind, und
(b) 5 bis 90 Gew.-%, bezogen auf das Gesamtgewicht des Gemisches aus Wasser und einem oder mehreren Lösemitteln, mindestens eines organischen Lösemittels, das das Polymer im Bereich der Anwendungstemperatur der Zusammensetzung vollständig zu lösen vermag und das teilweise oder vollständig mit Wasser mischbar ist und flüchtiger ist als Wasser.

2. Verwendung nach Anspruch 1, wobei das organische Lösemittel ausgewählt ist unter:
- den niederen Alkoholen mit 1 bis 4 Kohlenstoffatomen, wie Ethanol, Isopropanol und n-Propanol,
- den Ethern, wie Dimethoxyethan,
- den Ketonen, wie Aceton und Methylethylketon, und
- den niederen Carbonsäureestern mit 1 bis 3 Kohlenstoffatomen, wie Methylacetat und Ethylacetat,

3. Verwendung einer wässerigen Lösung oder einer wässerigen Dispersion als Mittel zum Überziehen keratinischer Materialien in einer kosmetischen Zusammensetzung, wobei die wässerige Lösung oder die wässerige Dispersion enthält:
(a) 0,1 bis 50 Gew,-%, bezogen auf das Gesamtgewicht der Zusammensetzung, mindestens eines nicht vernetzten Polymers, das im Bereich der Anwendungstemperatur der Zusammensetzung nach dem Trocknen zur Bildung einer Abscheidung auf einem keratinischen Träger befähigt ist und eine kritische Lösungstemperatur Tk in Wasser vom Typ LCST im Bereich von 0 bis 100°C aufweist und das unter den nicht vernetzten Homopolymeren oder Copolymeren von Monomeren, die eine Amidgruppe aufweisen, ausgewählt ist, die unter den Poly(N-isopropylacrylamid)-Homopolymeren, den Copolymeren von N-Isoprypylacrylamid und einem C₁₋₁₈-(Meth)acrylsäureester (beispielsweise Butylacrylat), den Copolymeren von N-Isopropylacrylamid und Methacrylsäure, und den Polymeren oder Copolymeren von Vinylcaprolactam ausgewählt sind, und
(b) mindestens einen grenzflächenaktiven Stoff, der 4 bis 30 Gew.-%, vorzugsweise 10 bis 25 Gew.-% und noch bevorzugter 12 bis 20 Gew.-% des Gesamtgewichts der am Ende vorliegenden Zusammensetzung ausmacht, und/oder mindestens ein hydrophiles Polymer, das keine kritische Temperatur Tk in dem oben angegebenen Bereich aufweist und befähigt ist, mit dem genannten Polymer in physikalische Wechselwirkungen zu treten, wobei es in der Zusammensetzung in einer Konzentration im Bereich von 5 bis 50 Gew.-% und vorzugsweise von 10 bis 30 Gew.-%, bezogen auf das Gesamtgewicht sämtlicher in der Zusammensetzung enthaltener Polymere, vorliegt.

4. Verwendung nach Anspruch 3, wobei das hydrophile Polymer ausgewählt ist unter:
- den Polyvinylalkoholen sowie ihren Copolymeren,
- den Polysacchariden oder den Cellulosepolymeren,
- den natürlichen Proteinen oder den synthetischen Polypeptiden,
- den synthetischen Polymeren von Monomeren mit Amidgruppen und
- den Copolymeren auf der Basis von Monomeren mit anionischen Gruppen, vorzugsweise Carbonsäure-, Sulfonsäureoder Phosphonsäure-Gruppen.

5. Verwendung nach Anspruch 3, wobei der grenzflächenaktive Stoff allein oder im Gemisch unter anionischen, amphoteren, nichtionischen, zwitterionischen und kationischen grenzflächenaktiven Stoffen ausgewählt ist.

6. Kosmetische oder dermatologische Zusammensetzung, **dadurch gekennzeichnet, dass** sie als Mittel zum Überziehen keratinischer Materialien zumindest eine wässerig-organische Lösung enthält, die aufweist:
(a) 0,1 bis 50 Gew.-%, bezogen auf das Gesamtgewicht der Zusammensetzung, mindestens eines nicht vernetzten Polymers, das nach dem Trocknen zur Bildung einer Abscheidung oder eines Films auf einem keratinischen Träger befähigt ist und eine kritische Lösungstemperatur Tk in Wasser vom Typ LCST im Bereich von 0 bis 100 °C aufweist und das unter den nicht vernetzten Homopolymeren oder Copolymeren von Monomeren, die eine Amidgruppe aufweisen, ausgewählt ist, die unter den Poly(N-substituierten Acrylamiden), wie den Poly(N-isopropylacrylamid)-Homopolymeren, den Copolymeren von N-Isopropylacrylamid und einem C₁₋₁₈-(Meth)acrylsäureester (beispielsweise Butylacrylat) und den Copolymeren von N-Isopropylacrylamid und Methacrylsäure ausgewählt sind, und
(b) 5 bis 90 Gew.-%, bezogen auf das Gesamtgewicht des Gemisches aus Wasser und einem oder mehreren Lösemitteln, mindestens eines organischen Lösemittels, das das Polymer im Bereich der Anwendungstemperatur der Zusammensetzung vollständig zu lösen vermag und das teilweise oder vollständig mit Wasser mischbar ist und flüchtiger ist als Wasser.

7. Zusammensetzung nach Anspruch 6, worin die organischen Lösemittel in Konzentrationen von 10 bis 70 Gew.-%, bezogen auf das Gesamtgewicht des Gemisches Wasser/organische(s) Lösemittel, vorliegen.

8. Zusammensetzung nach einem der Ansprüche 6 bis 7, worin das organische Lösemittel ausgewählt ist unter:
- den niederen Alkoholen mit 1 bis 4 Kohlenstoffatomen, wie Ethanol, Isopropanol und n-Propanol,
- den Ethern, wie Dimethoxyethan,
- den Ketonen, wie Aceton und Methylethylketon, und
- den niederen Carbonsäureestern mit 1 bis 3 Kohlenstoffatomen, wie Methylacetat und Ethylacetat.

9. Kosmetische oder dermatologische Zusammensetzung, **dadurch gekennzeichnet, dass** sie als Mittel zum Überziehen keratinischer Materialien zumindest eine wässerige Lösung oder eine wässerige Dispersion enthält, die aufweist:
(a) 0,1 bis 50 Gew.-%, bezogen auf das Gesamtgewicht der Zusammensetzung, mindestens eines nicht vernetzten Polymers, das im Bereich der Anwendungstemperatur der Zusammensetzung nach dem Trocknen zur Bildung einer Abscheidung auf einem keratinischen Träger befähigt ist und eine kritische Lösungstemperatur Tk in Wasser vom Typ LCST im Bereich von 0 bis 100 °C aufweist und das unter den nicht vernetzten Homopolymeren oder Copolymeren von Monomeren, die eine Amidgruppe aufweisen, ausgewählt ist, die unter den Poly(N-isopropylacrylamid)-Homopolymeren, den Copolymeren von N-Isopropylacrylamid und einem C₁₋₁₈-(Meth)acrylsäureester (beispielsweise Butylacrylat), den Copolymeren von N-Isopropylacrylamid und Methacrylsäure und den Polymeren oder Copolymeren von Vinylcaprolactam ausgewählt sind, und
(b) mindestens einen grenzflächenaktiven Stoff, der 4 bis 30 Gew.-%, vorzugsweise 10 bis 25 Gew.-% und noch bevorzugter 12 bis 20 Gew.-% des Gesamtgewichts der am Ende vorliegenden Zusammensetzung ausmacht, und/oder mindestens ein hydrophiles Polymer, das keine kritische Temperatur Tk in dem oben angegebenen Bereich aufweist und befähigt ist, mit dem genannten Polymer in physikalische Wechselwirkungen zu treten, wobei es in der Zusammensetzung in einer Konzentration im Bereich von 5 bis 50 Gew.-% und vorzugsweise von 10 bis 30 Gew.-%, bezogen auf das Gesamtgewicht sämtlicher in der Zusammensetzung enthaltener Polymere, vorliegt.

10. Zusammensetzung nach Anspruch 9, worin das hydrophile Polymer ausgewählt ist unter:
- den Polyvinylalkoholen sowie ihren Copolymeren,
- den Polysacchariden oder den Cellulosepolymeren,
- den natürlichen Proteinen oder den synthetischen Polypeptiden,
- den synthetischen Polymeren von Monomeren mit Amidgruppen und
- den Copolymeren auf der Basis von Monomeren mit anionischen Gruppen, vorzugsweise Carbonsäure-, Sulfonsäureoder Phosphonsäure-Gruppen.

11. Zusammensetzung nach einem der Ansprüche 6 bis 10, worin die nicht vernetzten Polymere in Konzentrationen von 0, 5 bis 40 Gew.-%, bezogen auf das Gesamtgewicht der Zusammensetzung, vorliegen.

12. Zusammensetzung nach einem der Ansprüche 6 bis 11, **dadurch gekennzeichnet, dass** sie als Produkt für die Haare, insbesondere zum Waschen, zur Pflege, zum Konditionieren, für den Halt der Frisur oder für die Formgebung von Keratinfasern, verwendet wird.

13. Zusammensetzung nach einem der Ansprüche 6 bis 12, **dadurch gekennzeichnet, dass** sie als Zusammensetzung für die Haare, die nicht ausgespült wird, und insbesondere als Frisierprodukt, wie Wasserwellenlotionen oder Fönwellenlotionen, oder als Zusammensetzung zum Fixieren und zum Frisieren, wie Lacke oder Sprays, verwendet wird.

14. Zusammensetzung nach einem der Ansprüche 6 bis 12, **dadurch gekennzeichnet, dass** sie als Zusammensetzung für die Haare, die ausgespült wird, und insbesondere als Haarwaschmittel mit frisurgestaltenden und/oder konditionierenden Eigenschaften oder als Mittel zur Anwendung nach der Haarwäsche verwendet wird.

15. Zusammensetzung nach einem der Ansprüche 6 bis 11, **dadurch gekennzeichnet, dass** sie als Pflegeprodukt und/ oder Hygieneprodukt verwendet wird.

16. Zusammensetzung nach einem der Ansprüche 6 bis 11, **dadurch gekennzeichnet, dass** sie als Mittel zum Abschminken verwendet wird.

17. Zusammensetzung nach einem der Ansprüche 6 bis 11, **dadurch gekennzeichnet, dass** sie als Schminkprodukt verwendet wird.

18. Zusammensetzung nach Anspruch 17, **dadurch gekennzeichnet, dass** sie als Nagellack oder als Pflegegrundmasse für die Nägel verwendet wird.

19. Verfahren zur nichttherapeutischen kosmetischen Behandlung der Haut, der Kopfhaut, der Haare, der Wimpern, der Augenbrauen, der Nägel oder der Schleimhäute, **dadurch gekennzeichnet, dass** eine Zusammensetzung nach einem der Ansprüche 6 bis 18 gemäß dem herkömmlichen Anwendungsverfahren dieser Zusammensetzung auf den keratinischen Träger aufgebracht wird.

## Claims

1. use, as agent for coating keratinous substances, in a cosmetic composition, of an aqueous/organic solution containing:
(a) 0.1 to 50% by weight, with respect to the total weight of the composition, of at least one non-crosslinked polymer capable of forming a deposit or a film on a keratinous substrate after drying and exhibiting a critical temperature Tc for solubility in water of the LCST type ranging from 0° to 100°C, chosen from the group composed of non-crosslinked homopolymers or copolymers of monomers containing an amide group which are chosen from poly(N-substituted acrylamide)s, such as poly(N-isopropylacrylamide) homopolymers, copolymers of N-isopropylacrylamide and of C₁-C₁₈ ester of (meth)acrylic acid (for example, butyl acrylate) or copolymers of N-isopropylacrylamide and of methacrylic acid; and
(b) 5 to 90% by weight, with respect to the total weight of the water/solvent(s) mixture of at least one total organic solvent for the said polymer within the temperature range for use of the composition which is partially or completely miscible with water and more volatile than water.

2. Use according to Claim 1, in which the organic solvent is chosen from:
- C₁-C₄ lower alcohols, such as ethanol, isopropanol or n-propanol;
- ethers, such as dimethoxyethane;
- ketones, such as acetone or methyl ethyl ketone;
- C₁-C₃ lower carboxylic acid esters, such as methyl acetate or ethyl acetate.

3. Use, as agent for coating keratinous substances, in a cosmetic composition, of an aqueous solution or of an aqueous dispersion containing:
(a) 0.1 to 50% by weight, with respect to the total weight of the composition, of at least one non-crosslinked polymer capable of forming, within the temperature range for use of the composition, a deposit on a keratinous substrate after drying and exhibiting a critical temperature Tc for solubility in water of the LCST type ranging from 0° to 100°C chosen from the group composed of non-crosslinked homopolymers or copolymers of monomers containing an amide group which are chosen from poly(N-isopropylacrylamide) homopolymers, copolymers of N-isopropylacrylamide and of C₁-C₁₈ ester of (meth) acrylic acid (for example, butyl acrylate) or copolymers of N-isopropylacrylamide and of methacrylic acid; or polymers or copolymers of vinylcaprolactam;
and
(b) at least one surface-active agent, representing 4 to 30% by weight, preferably from 10 to 25% by weight, more preferably from 12 to 20% by weight, of the total weight of the final composition, and/or at least one hydrophilic polymer not exhibiting a critical temperature Tc within the range indicated above, capable of establishing physical interactions with the said polymer, present in the composition at a concentration ranging from 5 to 50% by weight, preferably from 10 to 30% by weight, with respect to the total weight of all the polymers present in the composition.

4. Use according to Claim 3, in which the hydrophilic polymer is chosen from the group composed of:
- poly(vinyl alcohol)s and their copolymers;
- polysaccharides or cellulose polymers;
- natural proteins or synthetic polypeptides;
- synthetic polymers of monomers containing amide groups;
- copolymers based on monomers containing anionic groups, preferably carboxyl, sulphonic or phosphonic groups.

5. Use according to Claim 3, in which the surface-active agent is chosen, alone or as a mixture, from anionic, amphoteric, non-ionic, zwitterionic and cationic surfactants.

6. Cosmetic or dermatological composition, **characterized in that** it contains at least, as agent for coating keratinous substances, one aqueous/organic solution containing:
(a) 0.1 to 50% by weight, with respect to the total weight of the composition, of at least one non-crosslinked polymer capable of forming a deposit or a film on a keratinous substrate after drying and exhibiting a critical temperature Tc for solubility in water of the LCST type ranging from 0° to 100°C, chosen from the group composed of non-crosslinked homopolymers or copolymers of monomers containing an amide group which are chosen from poly(N-substituted acrylamide)s, such as poly(N-isopropylacrylamide) homopolymers, copolymers of N-isopropylacrylamide and of C₁-C₁₈ ester of (meth)acrylic acid (for example, butyl acrylate) or copolymers of N-isopropylacrylamide and of methacrylic acid; and
(b) 5 to 90% by weight, with respect to the total weight of the water/solvent(s) mixture, of at least one total organic solvent for the said polymer within the temperature range for use of the composition which is partially or completely miscible with water and more volatile than water.

7. Composition according to Claim 6 in which the organic solvents are present in concentrations ranging from 10 to 70% by weight with respect to the total weight of the water/organic solvent(s) mixture.

8. Composition according to either of Claims 6 and 7, in which the organic solvent is chosen from:
- C₁-C₄ lower alcohols, such ethanol, isopropanol or n-propanol;
- ethers, such as dimethoxyethane;
- ketones, such as acetone or methyl ethyl ketone;
- C₁-C₃ lower carboxylic acid esters, such as methyl acetate or ethyl acetate.

9. Cosmetic or dermatological composition, **characterized in that** it contains at least, as agent for coating keratinous substances, one aqueous solution or one aqueous dispersion containing:
(a) 0.1 to 50% by weight, with respect to the total weight of the composition, of at least one non-crosslinked polymer capable of forming, within the temperature range for use of the composition, a deposit on a keratinous substrate after drying and exhibiting a critical temperature Tc for solubility in water of the LCST type ranging from 0° to 100°C, chosen from the group composed of non-crosslinked homopolymers or copolymers of monomers containing an amide group which are chosen from poly(N-isopropylacrylamide) homopolymers, copolymers of N-isopropylacrylamide and of C₁-C₁₈ ester of (meth)acrylic acid (for example, butyl acrylate) or copolymers of N-isopropylacrylamide and of methacrylic acid; or polymers or copolymers of vinylcaprolactam;
and
(b) at least one surface-active agent, representing 4 to 30% by weight, preferably from 10 to 25% by weight, more preferably from 12 to 20% by weight, of the total weight of the final composition, and/or at least one hydrophilic polymer not exhibiting a critical temperature Tc within the range indicated above, capable of establishing physical interactions with the said polymer, present in the composition at a concentration ranging from 5 to 50% by weight, preferably from 10 to 30% by weight, with respect to the total weight of all the polymers present in the composition.

10. Composition according to Claim 9, in which the hydrophilic monomer is chosen from the group composed of:
- poly(vinyl alcohol)s and their copolymers;
- polysaccharides or cellulose polymers;
- natural proteins or synthetic polypeptides;
- synthetic polymers of monomers containing amide groups;
- copolymers based on monomers containing anionic groups, preferably carboxyl, sulphonic or phosphonic groups.

11. Composition according to one of Claims 6 to 10, in which the non-crosslinked polymers are present in concentrations ranging from 0.5 to 40% by weight with respect to the total weight of the composition.

12. Composition according to one of Claims 6 to 11, **characterized in that** it is used as hair product, in particular for washing, caring for, conditioning or form retention of the hairstyle or shaping keratinous fibres.

13. Composition according to one of Claims 6 to 12, **characterized in that** it is used as leave-in hair composition, in particular as styling products, such as hair-setting lotions, blow-drying lotions or fixing and styling compositions, such as lacquers or sprays.

14. Composition according to one of Claims 6 to 12, **characterized in that** it is used as rinse-out hair composition, in particular as styling and/or conditioning shampoo or as conditioner.

15. Composition according to any one of Claims 6 to 11, **characterized in that** it is used as care and/or hygiene product.

16. Composition according to any one of Claims 6 to 11, **characterized in that** it is used as make-up removal product.

17. Composition according to any one of Claims 6 to 11, **characterized in that** it is used as make-up product.

18. Composition according to Claim 17, **characterized in that** it is used as nail varnish or nail care base.

19. Process for the non-therapeutic cosmetic treatment of the skin, scalp, hair, eyelashes, eyebrows, nails or mucous membranes, **characterized in that** a composition as defined according to any one of Claims 6 to 18 is applied on the keratinous substrate according to the usual technique for the use of this composition.
